# EUROPEAN PATENT SPECIFICATION

(11) **EP 4 027 792 B1**
(45) Date of publication and mention of the grant of the patent: **27.03.2024**
(21) Application number: 20772202.6
(22) Date of filing: 26.08.2020
(51) Int. Cl.: A01N 43/76, A01N 31/02, A01N 35/04, A01N 37/10, A01P 1/00

(54) **TOPICAL COMPOSITIONS**
TOPISCHE ZUSAMMENSETZUNGEN
COMPOSITIONS TOPIQUES

(30) Priority: 11.09.2019 EP 19196622
(43) Date of publication of application: 20.07.2022
(73) Proprietor: DSM IP Assets B.V., 6411 TE Heerlen (NL)
(72) Inventor: MENDROK-EDINGER, Christine, 4303 Kaiseraugst (CH)
(74) Representative: dsm-firmenich IP
(86) International application number: PCT/EP2020/073790
(87) International publication number: WO 2021/047905

(56) References cited:
- WO-A1-02/39972
- WO-A1-2019/180042
- US-A- 6 123 953

## Description

The present invention relates cosmetic compositions comprising a mixture of 1,4-di(benzoxazol-2'-yl)benzene and a preservative as well as to the use of said mixture as antimicrobial agent.

Microorganisms lead to the decay of food and goods of daily use such as cosmetic products. The reduction of growth of microorganisms is thus essential to enhance shelf life (storage stability) of the product itself but also to assure consumers safety. Thus, generally a preservative is added to the respective products. As the use of certain preservatives is, however, under strong public discussion, the option of being able to reduce or even avoid such agents is a strong desire in the industries, in particular in the cosmetic industry, as topical compositions such as cremes and lotions, due to a relatively high water content, are particular prone to be attacked by microorganism. Thus, there is an ongoing need for alternatives to help the cosmetic formula remain safe and prevent alteration in time.

Surprisingly, it has now been found that 1,4-di(benzoxazol-2'-yl)benzene is able to synergistically enhance the antimicrobial action of preservatives (often also referred to as antimicrobial agents) commonly used in cosmetic compositions. This surprising finding is very advantageous as it allows to the reduce the amount of preservatives in cosmetic compositions while, nevertheless, maintaining the antimicrobial properties necessary to assure an appropriate shelf life and consumer safety.

Thus, in a first embodiment the present invention relates to topical compositions comprising 1,4-di(benzoxazol-2'-yl)benzene and at least one compound selected from the group of hydroxyacetophenone, 1,3-propandiol and benzoic acid or a salt thereof.

In a second embodiment, the present invention relates to the use of a mixture of 1,4-di(benzoxazol-2'-yl)benzene and at least one compound selected from the group of hydroxyacetophenone, 1,3-propandiol and benzoic acid or a salt thereof as a synergistic antimicrobial agent mixture (preservation booster).

In third embodiment, the invention relates to the use of a mixture of 1,4-di(benzoxazol-2'-yl)benzene and at least one compound selected from the group of hydroxyacetophenone, 1,3-propandiol and benzoic acid or a salt thereof as antimicrobial agent, i.e. an agent which exhibits an antimicrobial activity, in particular to enhance shelf-life and/ or storage stability of a topical composition.

In particular the present invention is directed to the use of a mixture of 1,4-di(benzoxazol-2'-yl)benzene and at least one compound selected from the group of hydroxyacetophenone, 1,3-propandiol and benzoic acid or a salt thereof as antifungal and/ or anti-bacterial agent, more in particular as an agent for killing and/ or inhibiting the growth of fungi such as in particular yeast or molds and/ or bacteria (gram-positive and/ or gram negative), most preferably of Escherichia Coli (E. Coli), Pseudomonas aeruginosa, (P. aeruginosa), Staphylococcus aureus (S. aureus), Aspergillus brasiliensis (A. brasiliensis), and/ or Candida albicans (C. albicans) as well as mixtures thereof, in particular to enhance shelf-life and/ or storage stability of a topical composition.

In a further embodiment, the invention relates to a method for killing and/ or inhibiting growth of microbial cells, in particular of fungi such as yeast and/ or molds and/ or bacterial cells, most in particular of E. Coli, P. aeruginosa, S. aureus, A. brasiliensis, and/ or C. albicans as well as mixtures thereof, said method comprising contacting said microbial cells with a mixture of 1,4-di(benzoxazol-2'-yl)benzene and at least one compound selected from the group of hydroxyacetophenone, 1,3-propandiol and benzoic acid or a salt thereof.

In another embodiment, the present invention relates a method of inhibiting or delaying microbial breakdown of a topical composition, wherein said method comprises the step of adding to the topical composition an effective amount of 1,4-di(benzoxazol-2'-yl)benzene and at least one compound selected from the group of hydroxyacetophenone, 1,3-propandiol and benzoic acid or a salt thereof.

The methods and uses according to the present invention are non-therapeutic. The term 'effective amount' as used herein refers to an amount necessary to obtain the desired antimicrobial effect. The effective amount may, of course, vary depending upon known factors, such as in particular the specifics of the cosmetic formula, but can be adjusted by a person skilled in the art within the amounts and ratios as specified herein.

The term "antimicrobial activity" (or "antimicrobial effect") as used herein means a capability of killing and/or inhibiting the growth of microbial cells such as in particular bacterial and fungal cells (in particular mold and/ or yeast), for example, P. acnes, S. epidermis, M. furfur, A. brasiliensis, E. Coli, P. aeruginosa, S. aureus, A. brasiliensis, and/ or C. albicans as well as mixtures thereof, preferably, however, of E. Coli, P. aeruginosa, S. aureus, A. brasiliensis, and/ or C. albicans as well as mixtures thereof 1,4-Di(benzoxazol-2'-yl)benzene [CAS 904-39-2] is a colorless to light yellow, solid, which can be prepared as e.g. outlined in WO200239972 or illustrated in the example.

Due to its low solubility, in all embodiments of the present invention, the 1,4-di(benzoxazol-2'-yl)benzene is preferably used in micronized form, i.e. in the form of solid particles having a mean particle size of less than 1µm.

In a particular advantageous embodiment, said micronized form exhibits a mean particle size of at most 300 nm. Even more preferably, the micronized 1,4-di(benzoxazol-2'-yl)benzene exhibits a mean particle size selected in the range from 50 to 300 nm, more preferably in the range from 100 to 300 nm, most preferably in the range from 120 to 280 nm, such as in the range from 140 to 240 nm or in the range from 150 to 220 nm.

The term 'mean particle size' as used herein refers to the mean number-based particle size distribution Dₙ50 (also known as Dₙ0.5) as determined by laser diffraction e.g. with a Malvern Mastersizer 2000 (ISO 13320:2009).

The micronized 1,4-di(benzoxazol-2'-yl)benzene according to the present invention can be produced by standard micronization methods in the art as e.g. outlined in WO9522959 and WO9703643.

The micronized 1,4-di(benzoxazol-2'-yl)benzene according to the present invention may be used in powder form or as a dispersion thereof.

The micronized 1,4-di(benzoxazol-2'-yl)benzene according to the present invention can furthermore be in a crystalline or in a solid amorphous form. In all embodiments of the present invention it is preferred, that the micronized 1,4-di(benzoxazol-2'-yl)benzene is in a solid amorphous form.

The term "solid amorphous form," as used herein, refers to solid particles which are formed by fast formation/separation of a solid phase from a liquid phase in a solution or a mixture, so that the solid has no time to selectively form a crystal network and thus the obtained solid is in a predominantly disordered form (also referred to herein as 'solid amorphous 1,4-di(benzoxazol-2'-yl)benzene'), which form can be unambiguously identified by XRPD analysis by not exhibiting a base line separation of the peaks at 25-28 °2Theta (Cu K-alpha Radiation) measured using a Bruker D8 Advance powder X-ray diffractometer in reflection (Bragg-Brentano) geometry with a LynxEye detector and Cu Kα radiation.

The solid amorphous form of 1,4-di(benzoxazol-2'-yl)benzene according to the present invention is preferably prepared by a process encompassing the steps of (1) reacting terephthalic acid and 2-aminophenol to form 1,4-di(benzoxazol-2'-yl)benzene and (2) precipitating the thus obtained 1,4-di(benzoxazol-2'-yl)benzene, preferably in the presence of (ice) water to obtain solid amorphous 1,4-di(benzoxazol-2'-yl)benzene, which, in a consecutive step, is micronized according to standard methods in the art. Optionally, the process can contain intermediate purification steps. Alternatively the solid amorphous 1,4-di(benzoxazol-2'-yl)benzene can be further purified by washing with a solvent, in which the 1,4-di(benzoxazol-2'-yl)benzene is only hardly or not soluble such as e.g. toluene and/ or an alcohol such as 1-butanol without being limited thereto.

It is furthermore advantageous that, in all embodiments of the present invention, the micronized 1,4-di(benzoxazol-2'-yl)benzene is used in form of an aqueous dispersion thereof as this eases the incorporation into the topical compositions.

The amount of the 1,4-di(benzoxazol-2'-yl)benzene in such aqueous dispersions is preferably selected in the range from 10 to 90 wt.-%, 20 to 80 wt.-% or 30 to 70 wt-%, more preferably in the range from 25 to 60 wt.-%, most preferably in the range from 25 to 55 wt.-%, such as in the range from 25 to 50 wt.-%, in the range from 25 to 40 wt.-% or in the range from 25 to 35 wt.-%, based on the total weight of the aqueous dispersion.

The aqueous dispersion may furthermore contain one or more additives e.g. to facilitate micronization and/ or to stabilize the aqueous dispersion e.g. against sedimentation.

If present, the amount (total) of the one or more additives in the aqueous dispersion is preferably selected in the range from 0.01 to 25 wt.-%, more preferably in the range from 0.1 to 20 wt.-%, most preferably in the range from 0.5 to 15 wt.-%, such as in the range from 1 to 10 wt.-%, based on the total weight of the aqueous dispersion.

In all embodiments according to the present invention, the at least one additive is preferably selected from the group of surface-active ingredients such as in particular anionic, non-ionic, amphoteric and cationic surfactants, anti-foam agents, salts, wetting agents and thickeners as well as mixtures thereof.

Particularly suitable surface-active ingredients to be used in the aqueous dispersions are alkyl polyglucosides (APG's) having the generic formula CₙH₂₊ₙ O(C₆H₁₀O₅)ₓH, in which n is an integer selected in the range from 2 to 22 and x refers to the mean polymerization level of the glucoside moiety (mono-, di-, tri-, oligo-, and poly-glucoside). Such alkyl polyglucosides are e.g. commercially available under the tradename Green APG 0810 by Shanghai Fine Chemical (a C₈₋₁₀ alkyl poly-glucoside which is made from glucose derived from corn and C₈ and C₁₀ fatty alcohols derived from coconut and palm kernel oils) or Plantacare 1200 UP from BASF (a C12-C16 fatty alcohol polyglycoside). If present, the surface-active ingredient(s) are preferably used in an amount (total) selected in the range from 1 to 20 wt.-%, more preferably from 5 to 15 wt.-%, most preferably from 7 to 12.5 wt.-%, based on the total weight of the aqueous dispersion.

Particularly suitable anti-foam agents to be used in the aqueous dispersions are silicone oils such as in particular polydimethylsiloxanes and/ or silicon anti-foam agents such as in particular anhydrous dispersions of pyrogenic or hydrophobized silica in silicone oils. Most preferably the anti-foam agent is simethicone. If present, such anti-foam agent(s) are preferably used in an amount (total) selected in the range from 0 to 1 wt.-%, more preferably in an amount of 0.01 to 0.2 wt.-%, based on the total weight of the aqueous dispersion.

Suitable salts to be used in the aqueous dispersions include alkali and earth alkaline salts of phosphate, hydroxide such as e.g. disodium hydrogen phosphate and/ or sodium hydroxide. If present, the salt or mixtures thereof (total amount) are preferably used in amounts from 0.01 to 5 wt.-, more preferably from 0.1 to 4 wt.-%, most preferably from 0.5 to 2.5 wt.-%, based on the total weight of the aqueous dispersion.

Particularly suitable thickeners to be used in the aqueous dispersions encompass xanthan gum, gellan gum and/ or carboxymethylcellulose. Most preferably the thickener is xanthan gum or gellan gum. If present, such thickener(s) are preferably used in an amount (total) selected in the range from 0.1 to 1 wt.-%, more preferably in the range from 0.1 to 0.5 wt.-%, based on the total weight of the aqueous dispersion.

Particularly suitable wetting agents to be used in the aqueous dispersions are (poly)propyleneglycol such as most preferably propyleneglycol. If present, the amount of such wetting agent(s) is preferably selected in the range from 0.1 to 1 wt.-%, more preferably in an amount of 0.2 to 0.6 wt.-%, based on the total weight of the aqueous dispersion.

In all embodiments according to the present invention preferably an aqueous dispersion consisting essentially of the micronized 1,4-di(benzoxazol-2'-yl)benzene, water and optionally at least one additive with all the definitions and preferences as given herein is used.

Even more advantageously, the aqueous dispersions consist essentially of
(i) 25-60 wt.-%, preferably 25-50 wt.-%, based on the total weight of the aqueous dispersion, of the micronized 1,4-di(benzoxazol-2'-yl)benzene with all the preferences and definitions as given herein,
(ii) 5 to 15 wt.-%, preferably 7 to 12.5 wt.-%, based on the total weight of the aqueous dispersion, of a C₈₋₁₆ alkyl polyglucoside,
(iii) 0 to 3 wt.-%, preferably 0 to 2.5 wt.-%, based on the total weight of the aqueous dispersion of at least one additive selected from the group of thickeners, wetting agents and/ or anti-foam agents, and
(iv) water Ad 100 wt.-%, based on the total weight of the aqueous dispersion.

The term 'consists essentially of' as used according to the present invention means that the amounts of the ingredients (i) to (iv) sum up to 100 wt.-%, which is adjusted with the amount of water (iv). It is, however, not excluded that small amount of impurities or additives may be present which are, for example, introduced via the respective raw materials of the ingredients.

Most advantageously, the aqueous dispersions contain as additives (iii) propyleneglycol, one thickener selected from xanthan gum or gellan gum and optionally simethicone.

In all embodiments of the present invention the topical compositions preferably comprise the 1,4-di(benzoxazol-2'-yl)benzene in an amount selected in the range from about 0.1 to 20 wt.-%, more preferably in the range from about 0.25 to 15 wt.-%, most preferably in the range from about 0.3 to 10 wt.-%, such as in the range from about 0.3 to 7.5 wt.-%, based on the total weight of the composition (and always based on the active). Further suitable ranges encompass 0.25 to 10 wt.-%, 0.25 to 7.5 wt.-%, 0.25 to 5 wt.-%, 1 to 7.5 wt.-% as well as 1 to 5 wt.-%.

The term hydroxyacetophenone refers to o-, m- or p-hydroxyacetophenone. Particularly preferred in all embodiments of the present invention is p-hydroxyacetophenone [CAS 99-93-4] which is also called 1-(4-hydroxyphenyl)-ethanone and which is e.g. commercially available at Symrise as SymSave^{®} H.

In all embodiments of the present invention, the topical compositions preferably comprise the hydroxyacetophenone in an amount selected in the range from 0.001 to 5 wt.-%, more preferably in the range from 0.01 to 4 wt-%, most preferably in the range from 0.1 to 3 wt-%, based on the total weight of the composition. Further preferred ranges are 0.005 to 4.5 wt-%, 0.05 to 4 wt-%, and 0.25 to 3 wt-%, based on the total weight of the composition.

Alkane diols such as in particular straight chain and branched C₁₋₁₀alkane diols are well known preservation boosters for cosmetic applications.

1,3-propandiol [CAS 504-63-2] is e.g. commercially available under the tradename Zemea^{™} Propandiol at DuPont Tate & Lyle Bioproducts.

In all embodiments of the present invention, the topical compositions preferably comprise 1,3-propandiol in an amount selected in the range from 0.01 to 6 wt.-%, more preferably in the range from 0.1 to 5 wt-%, most preferably in the range from 0.5 to 4 wt-%, based on the total weight of the composition. Further preferred ranges are 0.5 to 3.5 wt-% and 0.5 to 3 wt.-%, based on the total weight of the composition.

Benzoic acid as well as salts thereof are well known preservatives commonly used in foods, pharmaceuticals and cosmetics. Preferred salts are the potassium or sodium salts, most preferably the sodium salt. Particularly preferred in all embodiments of the present invention is the use of benzoic acid [CAS 65-85-0] and/ or sodium benzoate [CAS 532-32-1], most preferably of sodium benzoate which is e.g. commercially available at Emerald Kalama Chemicals under the trade name Purox^{®} S Grains.

In all embodiments of the present invention, the topical composition preferably comprises the benzoic acid or a salt thereof in an amount selected in the range from 0.001 to 6 wt.-%, more preferably in the range from 0.01 to 4 wt-%, most preferably in the range from 0.1 to 3 wt-%, based on the total weight of the composition. Further preferred ranges encompass 0.1 to 1 wt-% and 0.1 to 0.8 wt.-%, based on the total weight of the composition.

The weight ratio of 1,4-di(benzoxazol-2'-yl)benzene to the at least one compound selected from the group of hydroxyacetophenone, 1,3-propandiol and benzoic acid or a salt thereof is preferably selected in the range of between 40:1 to 1:40, more preferred between 30:1 to 1:30, even more preferred between 20:1 and 1:20 such as in the range of 15:1 to 1:15 or 15:1 to 1:10.

Most preferably, in case of hydroxyacetophenone, the weight-ratio is selected in the range of between 30:1 to 1:2, such as between 25:1 to 1:1.

Most preferably, in case of 1,3-propandiol the weight-ratio is selected in the range of between 15 to 1 to 1 to 5, such as between 10:1 to 1 to 1:3.

Most preferably, in case of benzoic acid or a salt thereof the weight-ratio is selected in the range of between 30:1 to 1:1, more preferably between 25:1 to 1:1.

In all embodiments, preferably, the weight-ratio of 1,4-di(benzoxazol-2'-yl)benzene to the at least one compound selected from the group of hydroxyacetophenone, 1,3-propandiol and benzoic acid or a salt thereof is ≥ 0.5, more preferably ≥ 0.75, most preferably ≥ 1.

If the compound is selected from hydroxyacetophenone and/ or the benzoic acid or a salt thereof, it is also advantageous to use the 1,4-di(benzoxazol-2'-yl)benzene in an excess, i.e. in an amount higher than the amount of the hydroxyacetophenone and/ or the benzoic acid or a salt thereof such as in a weight-ratio larger than 1, more preferably in a weight-ratio of at least 1.25, most preferably in a weight ratio of at least 2.

To make use of the synergistic anti-microbial activity of the mixture of 1,4-di(benzoxazol-2'-yl)benzene and at least one compound selected from the group of hydroxyacetophenone, 1,3-propandiol and benzoic acid or a salt thereof, it can be used in a multiplicity of formulations such as, for example, a lotion, a crème or a gel.

The amount of 1,4-di(benzoxazol-2'-yl)benzene employed according to the present invention is preferably selected in the range from 0.1 to 20 wt.-%, more preferably in the range from about 0.25 to 15 wt.-%, most preferably in the range from about 0.5 to 10 wt.-%, such as in the range from 0.3 to 7.5 wt.-% (always based on the active). Further suitable ranges encompass 0.25 to 10 wt.-%, 0.25 to 7.5 wt.-%, 0.25 to 5 wt.-%, 1 to 7.5 wt.-% as well as 1 to 5 wt.-%.

On the one hand, the use according to the present invention can be a technical use such as to reduce or prevent microbial growth in topical compositions, for example to enhance shelf-life and/ or storage stability. The use optionally encompasses the step of appreciating the effect compared to a respective product not containing1,4-di(benzoxazol-2'-yl)benzene.

On the other hand, the use according to the invention of the mixture of 1,4-di(benzoxazol-2'-yl)benzene and at least one compound selected from the group of hydroxyacetophenone, 1,3-propandiol and benzoic acid or a salt thereof can take place in a cosmetic or pharmaceutical sense. A pharmaceutical application is conceivable, for example, in the case of a specific anti-microbial therapy. A cosmetic application is conceivable, for example, for maintaining a healthy skin homeostasis and/ or for balancing the skin microbiome by reducing the number of unwanted microbes such as . Coli, P. aeruginosa, S. aureus, A. brasiliensis, and/ or C. albicans on the skin.

In all embodiments of the present invention, the use is preferably technical or cosmetic (non-therapeutic).

The topical compositions according to the present invention are preferably cosmetic, dermatological or pharmaceutical compositions which are topically applied to mammalian keratinous tissue such as in particular to human skin or the human scalp and hair. Particularly preferred topical compositions in all embodiments of the present invention are cosmetic compositions.

The term "cosmetic composition" as used in the present application refers to cosmetic compositions as defined under the heading "Kosmetika" in Römpp Lexikon Chemie, 10th edition 1997, Georg Thieme Verlag Stuttgart, New York as well as to cosmetic compositions as disclosed in A. Domsch, "Cosmetic Compositions", Verlag für chemische Industrie (ed. H. Ziolkowsky), 4th edition, 1992.

The topical compositions according to the present invention preferably further comprise a physiologically acceptable medium, that is to say a medium compatible with keratinous substances, such as the skin, mucosa, and keratinous fibers. Preferably, the physiologically acceptable medium is a cosmetically acceptable carrier.

The term cosmetically acceptable carrier refers to all carriers and/or excipients and/ or diluents conventionally used in cosmetic compositions such as in particular oils (cosmetic oils), water, surfactants, emulsifiers, thickeners.

The topical compositions according to the present invention are generally prepared by admixing the 1,4-di(benzoxazol-2'-yl)benzene and the at least one compound selected from the group of hydroxyacetophenone, 1,3-propandiol and benzoic acid or a salt thereof in the amounts indicated herein with a suitable carrier.

The exact amount of carrier will depend upon the actual level of the 1,4-di(benzoxazol-2'-yl)benzene and the at least one compound selected from the group of hydroxyacetophenone, 1,3-propandiol and benzoic acid or a salt thereof and any other optional ingredients that one of ordinary skill in the art would classify as distinct from the carrier (e.g., other active ingredients).

In an advantageous embodiment, the cosmetic or pharmaceutical compositions according to the present invention comprise from about 50% to about 99%, preferably from about 60% to about 98%, more preferably from about 70% to about 98%, such as in particular from about 80% to about 95% of a carrier, based on the total weight of the cosmetic composition.

In a particular advantageous embodiment, the carrier consists furthermore of at least 40 wt.-%, more preferably of at least 50 wt.-%, most preferably of at least 55 wt.-% of water, such as in particular of about 55 to about 90 wt.-% of water.

The compositions of the invention (including the carrier) may comprise conventional adjuvants and additives, such as preservatives/antioxidants, fatty substances/oils, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such compositions.

In accordance with the present invention, the compositions according to the invention may also comprise further cosmetically active ingredients conventionally used in cosmetic or pharmaceutical compositions. Exemplary active ingredients encompass skin lightening agents; UV-filters, agents for the treatment of hyperpigmentation; agents for the prevention or reduction of inflammation; firming, moisturizing, soothing, and/ or energizing agents as well as agents to improve elasticity and skin barrier.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the active ingredients as well as the excipients, diluents, adjuvants, additives etc. can, based on the desired product form and application, easily be determined by the skilled person. The additional ingredients can either be added to the oily phase, the aqueous phase or separately as deemed appropriate.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

Preferably, the topical compositions according to the invention are in the form of a suspension or dispersion in solvents or fatty substances, or alternatively in the form of an emulsion or micro emulsion (in particular of O/W- or W/O-type), PIT-emulsion, nano emulsion, multiple emulsion (e. g. O/W/O- or W/O/W-type), pickering emulsion, hydrogel, lipogel, one- or multiphase solution or vesicular dispersion.

In a preferred embodiment, the cosmetic compositions according to the present invention are emulsions and/ or gels. Even more preferably, the topical compositions are emulsions which contain an oily phase and an aqueous phase such as in particular O/W, W/O, Si/W, W/Si, O/W/O, W/O/W multiple or a pickering emulsions.

The amount of the oily phase (i.e. the phase containing all oils and fats including the polar oils) present in such emulsions is preferably at least 10 wt.-%, such as in the range from 10 to 60 wt.-%, preferably in the range from 15 to 50 wt.-%, most preferably in the range from 15 to 40 wt.-%, based on the total weight of the topical composition.

The amount of the aqueous phase present in such emulsions is preferably at least 20 wt.-%, such as in the range from 20 to 90 wt.-%, preferably in the range from 30 to 80 wt.-%, most preferably in the range from 30 to 70 wt.-%, based on the total weight of the topical composition.

The topical compositions in accordance with the invention can be in the form of a liquid, lotion, a thickened lotion, a gel, a cream, a milk, an ointment or a paste.

The topical compositions according to the invention have a pH in the range from 3-10, preferably in the range from pH of 3-8, most preferred in the range from pH 3-7.5. The pH is adjusted by methods known to a person skilled in the art, e.g. by using an acid such as a hydroxy acid including glycolic acid, lactic acid, malic acid, citric acid and tartaric acid or a base such as e.g. sodium or potassium hydroxide or ammonium hydroxide as well as mixtures thereof.

Preferably, in the compositions according to the invention the acid or base, if present, is used in an amount of at least 0.0001 wt.-%, such as e.g. in an amount of 0.01-1 wt.-%, in particular in an amount of 0.01 to 0.5 wt.-%.

The topical compositions according to the present invention may comprise an additional preservative or preservation booster. Particular suitable preservatives or preservation booster in all embodiments of the present invention are sorbic acid, potasssium sorbate, dehydroacetic acid, caprylhydroxamic acid, alcohol, alcohol denat., monoglycerides such as glyceryl laurate, propylene glycol caprylate, propylene glycol heptanoate as well as mixtures thereof. When present, the additional preservative and/ or preservation booster is preferably used in an amount of 0.01 to 2 wt.-%, more preferably in an amount of 0.05 to 1.5 wt.-%, most preferably in an amount of 0.1 to 1.0 wt.-%, based on the total weight of the composition.

It is furthermore preferred that the topical compositions according to the present invention are free of any parabenes, benzethoniumchlorid, piroctone olamine, lauroylarginat, methylisothiazolinon, chlormethylisothiazolinon, bronopol, benzalkoniumchloride, formaldeh releasing compounds, salicylic acid, triclosan, DMDM hydantoin, chlorphenesin and IPBC (lodopropinylbutyl carbamate).

It is furthermore preferred that topical compositions according to the present invention do not comprise a hyperbranched copolymer, such as in particular a hyperbranched of the monomers dodecenyl succinic acid anhydride, diisopropanol amine, bis-dimethylaminopropyl amine having terminal groups of formula

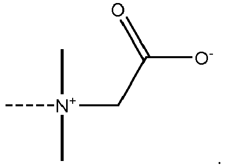

The topical compositions according to the present invention are in particular skin care preparations, functional preparations and/ or hair care preparations such as most in particularly skin care preparations.

Examples of skin care preparations are, in particular, light protective preparations (sunscreen preparations), anti-ageing preparations, preparations for the treatment of photo-ageing, body oils, body lotions, body gels, treatment creams, skin protection ointments, moisturizing preparations such as moisturizing gels or moisturizing sprays, face and/or body moisturizers, as well as skin lightening preparations.

Examples of functional preparations are cosmetic compositions containing active ingredients such as hormone preparations, vitamin preparations, vegetable extract preparations, anti-ageing preparations, and/or antimicrobial (antibacterial or antifungal) preparations without being limited thereto.

Examples of hair care preparations which are suitable according to the invention and which may be mentioned are shampoos, hair conditioners (also referred to as hair rinses, hairdressing compositions, hair tonics, hair regenerating compositions, hair lotions, water wave lotions, hair sprays, hair creams, hair gels, hair oils, hair pomades or hair brilliantines. Accordingly, these are always preparations which are applied to the hair and the scalp for a shorter or longer time depending on the actual purpose for which they are used.

The topical compositions according to the present invention are advantageously O/W emulsions, W/O emulsions and/ or gels.

Furthermore, the topical compositions in the form of O/W emulsions, W/O emulsions and/ or gels according to the present invention are skin care preparation intended for protecting the skin against the harmful effects of UV irradiation, for maintaining a healthy skin homeostasis and/ or for maintaining skin microbiome balance. Preferably in all embodiments of the present invention the skin care preparation is a sun care preparation.

It is preferred that said sun care preparation comprises at least one UV filter. The UV filter may be a liquid or solid UV filter. The UV filter may be a UV-A or UV-B filters. Suitable liquid UV-filter absorb light in the UVB (280 - 315 nm) and/ or UVA (315 - 400 nm) range and are liquid at ambient temperature (i.e. 25°C). Such liquid UV-filter are well known to a person in the art and encompass in particular cinnamates such as e.g. octyl methoxy¬cinnamate (PARSOL^{®} MCX) and isoamyl methoxycinnamate (Neo Heliopan^{®} E 1000), salicylates such as e.g. homosalate (3,3,5 trimethylcyclohexyl 2-hydroxybenzoate, PARSOL^{®} HMS) and ethylhexyl salicylate (also known as ethylhexyl salicylate, 2 ethylhexyl 2-hydroxybenzoate, PARSOL^{®} EHS), acrylates such as e.g. octocrylene (2 ethylhexyl 2-cyano-3,3-diphenylacrylate, PARSOL^{®} 340) and ethyl 2-cyano-3,3 diphenylacrylate, esters of benzalmalonic acid such as in particular dialkyl benzalmalonates such as e.g. di (2-ethylhexyl) 4-methoxy¬benz¬al¬malonate and polysilicone 15 (PARSOL^{®} SLX), dialkylester of naphthalates such as e.g. diethylhexyl 2,6-naphthalate (Corapan^{®} TQ), syringylidene malo¬na¬tes such as e.g. diethylhexyl syringylidene malonate (Oxynex^{®} ST liquid) as well as benzotriazolyl dodecyl p-cresol (Tinoguard^{®} TL) as well as benzophenone-3 and drometrizole trisiloxane.

Particular advantageous liquid UV-filter are octyl methoxycinnamate, homosalate, ethylhexyl salicylate, octocrylene, diethylhexyl 2,6-naphthalate, diethylhexyl syringylidene malonate, benzotriazolyl dodecyl p-cresol, benzo-phenone-3, drometrizole trisiloxane, Polysilicone-15 as well as mixtures thereof.

Suitable solid UV-filter absorb light in the UVB and/ or UVA range and are solid at ambient temperature (i.e. 25°C). They are particularly solid organic UV filters. Particularly suited solid UV-filters are of the group consisting of bis-ethylhexyl-oxyphenol methoxyphenyl triazine, butyl methoxydibenzoyl methane, methylene bis-benzotriazolyl tetramethylbutylphenol, diethylamino hydroxybenzoyl hexyl benzoate, ethylhexyl triazone, diethylhexyl butamido triazone, and 4-methyl¬benzyil¬dene camphor.

The amount of an individual organic UV filter is preferably in the range of 0.1 to about 10 % by weight, preferable in the range of 0.5 to 7.5 % by weight, most preferably in the range of 1 to 5 % by weight, based on the total weight of the sun care preparation.

In case of a sun care composition, the total amount of organic UV filter (s) depends strongly on the targeted UV protection of said composition and is typically in the range of between 1 to 50% by weight, preferably between 5 to 40% by weight, based on the total weight of said sun care preparation.

A sun care preparation with an SPF 15 (SPF=sun protection factor), for example, comprises preferably a total amount of organic UV filter (s) of between 4 to 20% by weight, more preferably between 7 and 15 % by weight, based on the total weight of said sun care preparation.

A sun care preparation with an SPF 30, for example, comprises preferably a total amount of organic UV filter (s) of between 10 to 40% by weight, more preferably between 15 and 25 % by weight, based on the total weight of said sun care preparation.

A sun care preparation with an SPF 50, for example, comprises preferably a total amount of organic UV filter (s) of between 15 to 50% by weight, more preferably between 20 and 40 % by weight, based on the total weight of said sun care preparation.

It has been observed that, when adding the 1,4-di(benzoxazol-2'-yl)benzene and the at least one compound selected from the group of hydroxyacetophenone, 1,3-propandiol and benzoic acid or a salt thereof to a topical composition, the antimicrobial action is increased significantly. In other words, by adding the synergistic antimicrobial agent mixture to a composition, a smaller number of microbes are observed to grow than without the use of 1,4-di(benzoxazol-2'-yl)benzene or the compound selected from the group of hydroxyacetophenone, 1,3-propandiol and benzoic acid or a salt thereof, i.e. with 1,4-di(benzoxazol-2'-yl)benzene or a hydroxyacetophenone, 1,3-propandiol or benzoic acid or a salt thereof alone. 1,4-di(benzoxazol-2'-yl)benzen has, hence, a synergistic effect on the antimicrobial action of a hydroxyacetophenone, 1,3-propandiol and benzoic acid or a salt thereof, particularly in a cosmetic composition.

It has been further observed that the antimicrobial action is particularly pronounced in aqueous systems. This is very advantageous as it is known that it is typically the water phase of a product which is most susceptible to microbial growth.

The following examples are provided to further illustrate the compositions and effects of the present invention.

### Example

### 1. Preparation of 1,4-di(benzoxazol-2'-yl)benzene:

A mixture of 702 g polyphosphoric acid and 4.28 ml methanesulfonic acid was heated to 90°C. 65 g terephthalic acid and 107 g 2-aminophenol were added. The mixture was stirred under inert atmosphere at 180°C for 8 hours and then transferred to ice water. The precipitated product was filtered and washed with water and acetic acid. The precipitate was dispersed in water and the pH adjusted to 8.0 with sodium hydroxide, filtered and washed with water. The crude product was suspended in a mixture of toluene and 1-butanol 3.3:1, stirred at 85°C for one hour, filtered, washed with diethyl ether, and dried resulting in coarse particles of solid amorphous 1,4-di(benzoxazol-2'-yl)benzene (in the following referred to as DBO).

### 2. Preparation of 1,4-di(benzoxazol-2'-yl)benzene, 30wt.-% aqueous dispersion:

Afterwards, a suspension of 175 g of the coarse particles, 324 g of water and 65 g Green APG 0810 was prepared and the suspension was milled for 2h with a LabStar laboratory mill using yttrium-stabilized zirconium oxide grinding beads (0.3mm, from Tosoh Ceramic, Japan) and cooling of the milling chamber (-12°C brine). After removal of the grinding beads, a 30% aqueous dispersion of micronized 1,4-di(benzoxazol-2'-yl)benzene (in the following referred to as DBO aq.) was obtained which was used in the antimicrobial tests and formulations as outlined below.

### 1. Antimicrobial activity

The antimicrobial efficacy was assessed in analogy to the regulatory challenge test method (NF EN ISO11930). Thus, mixtures of the respective active(s) with physiological serum with 0.85 wt.-% NaCl in the concentrations as outlined below are prepared under sterile conditions. Controls were also prepared under sterile conditions. The control as well as the mixtures were then deposed in 96-deep well plates (1.6 ml/well). The wells were contaminated with the respective bacterial or the fungal strains at 2.5*10⁵ to 5.6*10⁵ cfu/ml for the bacteria and 1*10⁴ to 2.5*10⁴ cfu/ml for the fungi to obtain the initial contamination as outlined below. After the contamination, each well was thoroughly mixed to ensure a homogeneous distribution of microbes. Then each plate was incubated at 22°C for 24h. The counting of the (remaining) population is carried out 24h after contamination. Then, the average log reduction is calculated [-log10{(value inoculum)/ (value sample)}] which is represented in tables 1-5.

**Table 1: Results using 0.3 wt.-% DBO**

| Microbe | inoculum | 24h | Log reduction |
|---|---|---|---|
| | colony count [cfu/ml] | | |
| Candida albicans (preser., yeast) | 32000 | 10000 | -0.51 |
| Aspergillus brasiliensis (preserv., mold) | 45000 | 400 | -2.05 |

**Table 2: Results using 1 wt.-% DBO aq. (0.3 wt.-% of active)**

| | inoculum | 24h | Log reduction |
|---|---|---|---|
| Candida albicans | 70000 | 40000 | -0.24 |
| Escherichia coli | 400000 | 10000 | -1.60 |
| Staphylococcus aureus | 550000 | 100000 | -0.74 |
| Pseudomonas aeruginosa | 330000 | 1000 | -2.52 |
| Aspergillus brasiliensis | 40000 | 1000 | -1.60 |

| | | | |
|---|---|---|---|
| *Δ = ({microorganism count t=0} - {microorganism count t = 24h}/ {microorganism count t=0})*100 | | | |

As can be seen in the tables above 1,4-di(benzoxazol-2'-yl)benzene has an antimicrobial effect over a broad range of microbes and can thus be used as preservation booster. Since it is known that typically the water phase of a product is most susceptible to microbial growth, 1,4-di(benzoxazol-2'-yl)benzene is thus particularly suitable to protect products comprising a water phase such as e.g. cosmetic compositions in the form of an O/W or W/O emulsion against microbial decay and breakdown and is thus suitable for improving its preservation.

**Table 3: Synergistic effect with p-Hydroxyacetophenone (HAP)**

| Test solution | Time [h] | Staphylococcus aureus colony count [cfu/ml] | Log reduction |
|---|---|---|---|
| 1.0 wt.-% DBO aq.* | 0 | 550000 | |
| | 24 | 100000 | -0.74 |
| 0.3 wt.-% HAP | 0 | 550000 | |
| | 24 | 70000 | -1.16 |
| 1.0 wt.-% DBO aq.* | 0 | 550000 | |
| 0.3 wt.-% HAP | 24 | 100 | -3.74 |
| Control | 0 | 550000 | |
| | 24 | 1000000 | +0.26 |

| | | | |
|---|---|---|---|
| *0.3 wt.-% active | | | |

As can be retrieved from table 2 DBO synergistically enhances the antimicrobial activity of p-Hydroxyacetophenone.

**Table 4: Synergistic effect with sodium benzoate**

| Test solution | Time [h] | Escherichia coli colony count [cfu/ml] | Log reduction |
|---|---|---|---|
| 1.0 wt.-% DBO aq.* | 0 | 400000 | |
| | 24 | 10000 | -1.60 |
| 0.2 wt.-% Sodium Benzoate | 0 | 400000 | |
| | 24 | 1000000 | +0.40 |
| 1.0 wt.-% DBO aq. | 0 | 400000 | |
| 0.2 wt.-% Sodium Benzoate | 24 | 100 | -3.60 |
| Control | 0 | 400000 | |
| | 24 | 1000000 | +0.40 |

| | | | |
|---|---|---|---|
| *0.3 wt.-% active | | | |

As can be retrieved from table 3 the combination of DBO and sodium benzoate exhibits a synergistic antimicrobial activity.

**Table 5: Synergistic effect with 1,3-propanediol (PDO)**

| Test solution | Time [h] | Candida albicans colony count [cfu/ml] | Log reduction |
|---|---|---|---|
| 1.0 wt.-% DBO* | 0 | 70000 | |
| | 24 | 40000 | -0.24 |
| 1.0 wt.-% PDO | 0 | 70000 | |
| | 24 | 40000 | -0.24 |
| 1.0 wt.-% DBO* | 0 | 70000 | |
| 1.0 wt.-% PDO | 24 | 10000 | -0.85 |
| Control | 0 | 70000 | |
| | 24 | 70000 | 0 |

| | | | |
|---|---|---|---|
| *0.3 wt.-% active | | | |

As can be retrieved from table 4 the combination of DBO and 1,3-propanediol exhibits a synergistic antimicrobial activity.

### 2. Formulation examples

### Sunscreen o/w

| Phase | INCI | Weight-% | | |
|---|---|---|---|---|
| A | PHENYLBENZIMIDAZOLE SULFONIC ACID | 0.5 | 0.5 | 0.5 |
| | AQUA | 10 | 10 | 10 |
| | TRIETHANOLAMINE | 0.4 | 0.4 | 0.4 |
| B | DISODIUM EDTA; AQUA | 0.1 | 0.1 | 0.1 |
| | AQUA | Add 100 | | |
| | BUTYLENE GLYCOL | 4 | 4 | 4 |
| | ACACIA SENEGAL GUM; XANTHAN GUM | 0.3 | 0.3 | 0.3 |
| | COCO-GLUCOSIDE; AQUA | 0.5 | 0.5 | 0.5 |
| | PROPANEDIOL | | | 3 |
| | SODIUM BENZOATE | 0.4 | | |
| C | C15-19 ALKANE | 5 | 5 | 5 |
| | OCTOCRYLENE | 6 | 6 | 6 |
| | POTASSIUM CETYL PHOSPHATE | 1.8 | 1.8 | 1.8 |
| | TOCOPHERYL ACETATE | 0.5 | 0.5 | 0.5 |
| | METHOXYDIBENZOYLMETHANE | 2.5 | 2.5 | 2.5 |
| | ETHYLHEXYL SALICYLATE | 5 | 5 | 5 |
| | TITANIUM DIOXIDE; SILICA; DIMETHICONE | 3 | 3 | 3 |
| | PHENOXYETHANOL; ETHYLHEXYLGLYCERIN | 1 | 1 | 1 |
| | DIISOPROPYL ADIPATE; PROPYLENE GLYCOL DICAPRYLATE/DICAPRATE; C12-15 ALKYL BENZOATE; DIISOPROPYL SEBACATE | 3 | 3 | 3 |
| D | SILICA | 1 | 1 | 1 |
| | DBO aq. | 1-3 | 1-3 | 1-3 |
| E | HYDROXYETHYL ACRYLATE/SODIUM ACRYLOYLDIMETHYL TAURATE COPOLYMER; POLYISOBUTENE; PEG-7 TRIMETHYLOLPROPANE COCONUT ETHER | 1 | 1 | 1 |
| | HYDROXYACETOPHENONE | | 0.75 | 0.6 |
| F | AQUA; SODIUM HYDROXIDE | 1.45 | 1.45 | 1.45 |

### Crème Gel

| Phase | INCI | Weight.-% | | |
|---|---|---|---|---|
| A | AQUA | Add 100 | | |
| | GLYCERIN | 3 | 3 | 3 |
| | PHENOXYETHANOL; ETHYLHEXYLGLYCERIN | 1 | | |
| | PROPANEDIOL | | | 3 |
| | SODIUM BENZOATE | | 0.5 | |
| B | CAPRYLIC/CAPRIC TRIGLYCERIDE | 5 | 5 | |
| | ETHYLHEXYL OLIVATE; SODIUM ACRYLATES COPOLYMER; POLYGLYCERYL-4 OLIVATE | 1.5 | 1.5 | |
| C | DBO aq. | 1-3 | 1-3 | 1-3 |
| | HYDROXYACETOPHENONE | 0.1 | | 0.7 |
| | SODIUM HYDROXIDE; AQUA | 0.05 | | 0.05 |
| | CITRIC ACID; AQUA | | 0.1 | |

### Sunscreen w/o

| Phase | INCI | Weight-% | | |
|---|---|---|---|---|
| A | AQUA | Add 100 | | |
| | PROPANEDIOL | 3.0 | | 3.0 |
| B | LAURYL PEG-10 TRIS(TRIMETHYLSILOXY)SILYLETHYL DIMETHICONE | 6.0 | 6.0 | 6.0 |
| | OCTOCRYLENE | 3.0 | 3.0 | 3.0 |
| | BUTYL METHOXYDIBENZOYLMETHANE | 2.0 | 2.0 | 2.0 |
| | POLYSILICONE-15 | 3.0 | 3.0 | 3.0 |
| | ZINC OXIDE; TRIETHOXYCAPRYLYLSILANE | 11.7 | 11.7 | 11.7 |
| | HOMOSALATE | 7.5 | 7.5 | 7.5 |
| | TITANIUM DIOXIDE; SILICA; DIMETHICONE | 3.0 | 3.0 | 3.0 |
| | DIMETHICONE | 3.0 | 3.0 | 3.0 |
| | ISONONYL ISONONANOATE | 10.0 | 10.0 | 10.0 |
| C | CYCLOPENTASILOXANE | 20.0 | 20.0 | 20.0 |
| | ALCOHOL DENAT. | 5.0 | 5.0 | 5.0 |
| | DBO aq. | 1-3 | 1-3 | 1-3 |
| | SODIUM BENZOATE | | 0.5 | |
| | HYDROXYACETOPHENONE | | | 0.2 |

### Skin Care O/W

| Phase | INCI | Weight-% | | |
|---|---|---|---|---|
| A | AQUA | Add 100 | | |
| | PROPANEDIOL | | | |
| | DISODIUM EDTA; AQUA | 0.05 | 0.05 | 0.05 |
| | XANTHAN GUM | 0.2 | 0.2 | 0.2 |
| | ETHOXYDIGLYCOL | 2.0 | 2.0 | 2.0 |
| | CETEARYL OLIVATE; SORBITAN OLIVATE | 4.0 | 4.0 | 4.0 |
| | DIMETHICONE | 2.0 | 2.0 | 2.0 |
| B | ISOPROPYL MYRISTATE | 4.0 | 4.0 | 4.0 |
| | DICAPRYLYL ETHER | 5.0 | 5.0 | 5.0 |
| | PARAFFINUM LIQUIDUM | 2.0 | 2.0 | 2.0 |
| | STEARIC ACID; PALMITIC ACID | 1.5 | 1.5 | 1.5 |
| | ISOSTEARYL ISOSTEARATE | 5.0 | 5.0 | 5.0 |
| C | DBO aq. | 1-3 | 1-3 | 1-3 |
| | SODIUM BENZOATE | 0.6 | | 4.0 |
| | HYDROXYACETOPHENONE | | 0.75 | 0.5 |
| | SODIUM HYDROXIDE; AQUA | 0.08 | 0.08 | 0.08 |

### Skin Care o/w

| Phase | INCI | Weight-% | | |
|---|---|---|---|---|
| A | AQUA | Add 100 | | |
| | HYDROXYPROPYL STARCH PHOSPHATE; AQUA | 1.0 | 1.0 | 1.0 |
| | HYDROXYACETOPHENONE | 0.6 | 0.5 | |
| | PROPANEDIOL | | 3.0 | |
| | SODIUM BENZOATE | | | 0.5 |
| | GLYCERIN | 2.0 | 2.0 | 2.0 |
| B | ISOPROPYL MYRISTATE | 2.0 | 2.0 | 2.0 |
| | CETEARYL ALCOHOL | 2.0 | 2.0 | 2.0 |
| | SQUALANE | 2.0 | 2.0 | 2.0 |
| | POTASSIUM CETYL PHOSPHATE | 3.0 | 3.0 | 3.0 |
| | COCOGLYCERIDES | 5.5 | 5.5 | 5.5 |
| | C12-15 ALKYL BENZOATE | 4.0 | 4.0 | 4.0 |
| | HYDROGENATED COCOGLYCERIDES | 4.0 | 4.0 | 4.0 |
| | PARAFFINUM LIQUIDUM | 3.0 | 3.0 | 3.0 |
| C | TOCOPHEROL | 0.2 | 0.2 | 0.2 |
| | SACCHARIDE ISOMERATE; AQUA; CITRIC ACID; SODIUM CITRATE | 1.0 | 1.0 | 1.0 |
| | ARGANIA SPINOSA KERNEL OIL | 2.0 | 2.0 | 2.0 |
| | DBO aq. | 1-3 | 1-3 | 1-3 |

### CC Cream o/w

| Phase | INCI | Weight-% | | |
|---|---|---|---|---|
| A | AQUA | Add 100 | | |
| | PROPANEDIOL | | 4.0 | 4.0 |
| | HYDROXYACETOPHENONE | 0.7 | 0.5 | |
| | SODIUM BENZOATE | | | 0.4 |
| B | MICROCRISTALLINE CELLULOSE ; CELLULOSE GUM | 2.0 | 2.0 | 2.0 |
| C | ETHYLHEXYL METHOXYCINNAMATE | 10.0 | 10.0 | 10.0 |
| | BEHENYL ALCOHOL | 4.0 | 4.0 | 4.0 |
| | GLYCERYL MYRISTATE | 4.0 | 4.0 | 4.0 |
| | ISOAMYL LAURATE | 14.0 | 14.0 | 14.0 |
| | OCTYLDODECANOL | 8.0 | 8.0 | 8.0 |
| | DIMETHICONE | 4.0 | 4.0 | 4.0 |
| | TITANIUM DIOXIDE; AQUA; GLYCERIN; PHENOXYETHANOL; CELLULOSE GUM; SODIUM POLYACRYLATE | 8.0 | 8.0 | 8.0 |
| | CI77499; AQUA; GLYCERIN; PHENOXYETHANOL; CELLULOSE GUM; SODIUM POLYACRYLATE | 0.25 | 0.25 | 0.25 |
| | CI77491; AQUA; GLYCERIN; PHENOXYETHANOL; CELLULOSE GUM; SODIUM POLYACRYLATE | 0.545 | 0.545 | 0.545 |
| | CI77492; AQUA; GLYCERIN; PHENOXYETHANOL; CELLULOSE GUM; SODIUM POLYACRYLATE | 2.2 | 2.2 | 2.2 |
| | TITANIUM DIOXIDE; SILICA; DIMETHICONE | 3.0 | 3.0 | 3.0 |
| | POTASSIUM CETYL PHOSPHATE | 3.0 | 3.0 | 3.0 |
| D | DBO aq. | 1-3 | 1-3 | 1-3 |

## Claims

1. A topical composition comprising comprising 1,4-di(benzoxazol-2'-yl)benzene and at least one compound selected from the group of hydroxyacetophenone, 1,3-propandiol and benzoic acid or a salt thereof, wherein the amount of 1,4-di(benzoxazol-2'-yl)benzene is selected in the range from 0.1 to 20 wt.-%, more preferably in the range from 0.25 to 15 wt.-%, most preferably in the range from about 0.3 to 10 wt.-%, based on the total weight of the composition.

2. The topical composition according to claim 1, wherein the amount of the hydroxyacetophenone is selected in the range from 0.001 to 5 wt.-%, preferably in the range from 0.01 to 4 wt-%, most preferably in the range from 0.1 to 3 wt-%, based on the total weight of the composition.

3. The topical composition according to anyone of the preceding claims, wherein the amount of the 1,3-propandiol is selected in the range from 0.01 to 5 wt.-%, preferably in the range from 0.1 to 4 wt-%, most preferably in the range from 0.5 to 3 wt-%, based on the total weight of the composition.

4. The topical composition according to anyone of the preceding claims, wherein the amount of the benzoic acid or a salt thereof is selected in the range from 0.001 to 6 wt.-%, preferably in the range from 0.01 to 4 wt-%, most preferably in the range from 0.1 to 3 wt-%, based on the total weight of the composition.

5. The topical composition according to anyone of the preceding claims, wherein the weight-ratio of the 1,4-di(benzoxazol-2'-yl)benzene to the at least one compound selected from the group of hydroxyacetophenone, 1,3-propandiol and benzoic acid or a salt thereof is selected in the range of between 50:1 to 1:10, preferably between 40:1 to 1:5, most preferably between 30:1 and 1:1.

6. The topical composition according to anyone of the preceding claims, wherein the hydroxyacetophenone is p-hydroxyacetophenone and the benzoic acid or a salt thereof is sodium benzoate.

7. The topical composition any one of the preceding claims, wherein the composition is an O/W emulsion, a W/O emulsion or a gel.

8. The topical composition according to any one of the preceding claims, wherein the composition comprises water and at least one agent selected from the group consisting of surfactants, emulsifiers, thickeners and oils.

9. A non-therapeutic topical method of treating the skin and/ or the scalp, said method comprising the steps of contacting the skin and/ or scalp with a cosmetic composition according to anyone of claim 1 to 8.

10. A method according to claim 9 for maintaining a healthy skin homeostasis and/ or for maintaining skin microbiome balance.

11. Use of a mixture of 1,4-di(benzoxazol-2'-yl)benzene and at least one compound selected from the group of hydroxyacetophenone, 1,3-propandiol and benzoic acid or a salt thereof as a non-therapeutic synergistic antimicrobial agent mixture.

12. The use according to claim 11, wherein the wherein the hydroxyacetophenone is p-hydroxyacetophenone and the benzoic acid or a salt thereof is sodium benzoate.

13. The use according to claim 11 or 12, to enhance shelf life and/ or storage stability of a topical composition.

14. A method of inhibiting or delaying microbial breakdown of a topical composition, wherein said method comprises the step of adding to the cosmetic composition an effective amount of 1,4-di(benzoxazol-2'-yl)benzene and at least one compound selected from the group of hydroxyacetophenone, 1,3-propandiol and benzoic acid or a salt thereof.

## Patentansprüche

1. Topische Zusammensetzung, umfassend 1,4-Di(benzoxazol-2'-yl)benzol und mindestens eine Verbindung, die aus der Gruppe Hydroxyacetophenon, 1,3-Propandiol und Benzoesäure oder einem Salz davon ausgewählt ist, wobei die Menge von 1,4-Di(benzoxazol-2'-yl)benzol im Bereich von 0,1 bis 20 Gew.-%, weiter bevorzugt im Bereich von 0,25 bis 15 Gew.-%, ganz besonders bevorzugt im Bereich von etwa 0,3 bis 10 Gew.-%, bezogen auf das Gesamtgewicht der Zusammensetzung, ausgewählt ist.

2. Topische Zusammensetzung nach Anspruch 1, wobei die Menge des Hydroxyacetophenons im Bereich von 0,001 bis 5 Gew.-%, vorzugsweise im Bereich von 0,01 bis 4 Gew.-%, ganz besonders bevorzugt im Bereich von 0,1 bis 3 Gew.-%, bezogen auf das Gesamtgewicht der Zusammensetzung, ausgewählt ist.

3. Topische Zusammensetzung nach einem der vorhergehenden Ansprüche, wobei die Menge des 1,3-Propandiols im Bereich von 0,01 bis 5 Gew.-%, vorzugsweise im Bereich von 0,1 bis 4 Gew.-%, ganz besonders bevorzugt im Bereich von 0,5 bis 3 Gew.-%, bezogen auf das Gesamtgewicht der Zusammensetzung, ausgewählt ist.

4. Topische Zusammensetzung nach einem der vorhergehenden Ansprüche, wobei die Menge der Benzoesäure oder eines Salzes davon im Bereich von 0,001 bis 6 Gew.-%, vorzugsweise im Bereich von 0,01 bis 4 Gew.-%, ganz besonders bevorzugt im Bereich von 0,1 bis 3 Gew.-%, bezogen auf das Gesamtgewicht der Zusammensetzung, ausgewählt ist.

5. Topische Zusammensetzung nach einem der vorhergehenden Ansprüche, wobei das Gewichtsverhältnis des 1,4-Di(benzoxazol-2'-yl)benzols zu der mindestens einen Verbindung, die aus der Gruppe Hydroxyacetophenon, 1,3-Propandiol und Benzoesäure oder einem Salz davon ausgewählt ist, im Bereich zwischen 50:1 bis 1:10, vorzugsweise zwischen 40:1 bis 1:5, ganz besonders bevorzugt zwischen 30:1 und 1:1, ausgewählt ist.

6. Topische Zusammensetzung nach einem der vorhergehenden Ansprüche, wobei es sich bei dem Hydroxyacetophenon um p-Hydroxyacetophenon handelt und es sich bei der Benzoesäure oder einem Salz davon um Natriumbenzoat handelt.

7. Topische Zusammensetzung nach einem der vorhergehenden Ansprüche, wobei es sich bei der Zusammensetzung um eine O/W-Emulsion, eine W/O-Emulsion oder ein Gel handelt.

8. Topische Zusammensetzung nach einem der vorhergehenden Ansprüche, wobei die Zusammensetzung Wasser und mindestens ein Mittel, das aus der Gruppe bestehend aus Tensiden, Emulgatoren, Verdickern und Ölen ausgewählt ist, umfasst.

9. Nichttherapeutisches topisches Verfahren zur Behandlung der Haut und/oder der Kopfhaut wobei das Verfahren die Schritte des Inkontaktbringens der Haut und/oder Kopfhaut mit einer kosmetischen Zusammensetzung nach einem der Ansprüche 1 bis 8 umfasst.

10. Verfahren nach Anspruch 9 zur Aufrechterhaltung einer gesunden Hauthomöostase und/oder zur Aufrechterhaltung der Hautmikrobiombalance.

11. Verwendung einer Mischung von 1,4-Di(benzoxazol-2'-yl)benzol und mindestens einer Verbindung, die aus der Gruppe Hydroxyacetophenon, 1,3-Propandiol und Benzoesäure oder einem Salz davon ausgewählt ist, als nichttherapeutische synergistische Mischung von antimikrobieller Mitteln.

12. Verwendung nach Anspruch 11, wobei es sich bei dem Hydroxyacetophenon um p-Hydroxyacetophenon handelt und es sich bei der Benzoesäure oder einem Salz davon um Natriumbenzoat handelt.

13. Verwendung nach Anspruch 11 oder 12 zur Verbesserung der Haltbarkeit und/oder Lagerstabilität einer topischen Zusammensetzung.

14. Verfahren zur Inhibierung oder Verzögerung des mikrobiellen Abbaus einer topischen Zusammensetzung, wobei das Verfahren den Schritt des Zugebens einer wirksamen Menge von 1,4-Di(benzoxazol-2'-yl)benzol und mindestens einer Verbindung, die aus der Gruppe Hydroxyacetophenon, 1,3-Propandiol und Benzoesäure oder einem Salz davon ausgewählt ist, zu der kosmetischen Zusammensetzung umfasst.

## Revendications

1. Composition topique comprenant du 1,4-di(benzoxazol-2'-yl)benzène et au moins un composé choisi dans le groupe de l'hydroxyacétophénone, du 1,3-propanediol et de l'acide benzoïque ou d'un sel correspondant, la quantité de 1,4-di(benzoxazol-2'-yl)benzène étant choisie dans la plage de 0,1 à 20 % en poids, plus préférablement dans la plage de 0,25 à 15 % en poids, le plus préférablement dans la plage d'environ 0,3 à 10 % en poids, sur la base du poids total de la composition.

2. Composition topique selon la revendication 1, la quantité de l'hydroxyacétophénone étant choisie dans la plage de 0,001 à 5 % en poids, préférablement dans la plage de 0,01 à 4 % en poids, le plus préférablement dans la plage de 0,1 à 3 % en poids, sur la base du poids total de la composition.

3. Composition topique selon l'une quelconque des revendications précédentes, la quantité du 1,3-propanediol étant choisie dans la plage de 0,01 à 5 % en poids, préférablement dans la plage de 0,1 à 4 % en poids, le plus préférablement dans la plage de 0,5 à 3 % en poids, sur la base du poids total de la composition.

4. Composition topique selon l'une quelconque des revendications précédentes, la quantité de l'acide benzoïque ou d'un sel correspondant étant choisie dans la plage de 0,001 à 6 % en poids, préférablement dans la plage de 0,01 à 4 % en poids, le plus préférablement dans la plage de 0,1 à 3 % en poids, sur la base du poids total de la composition.

5. Composition topique selon l'une quelconque des revendications précédentes, le rapport en poids du 1,4-di(benzoxazol-2'-yl)benzène sur l'au moins un composé choisi dans le groupe de l'hydroxyacétophénone, du 1,3-propanediol et de l'acide benzoïque ou d'un sel correspondant étant choisi dans la plage comprise entre 50 : 1 à 1 : 10, préférablement entre 40 : 1 et 1 : 5, le plus préférablement entre 30 : 1 et 1 : 1.

6. Composition topique selon l'une quelconque des revendications précédentes, l'hydroxyacétophénone étant la p-hydroxyacétophénone et l'acide benzoïque ou un sel correspondant étant le benzoate de sodium.

7. Composition topique selon l'une quelconque des revendications précédentes, la composition étant une émulsion H/E, une émulsion E/H ou un gel.

8. Composition topique selon l'une quelconque des revendications précédentes, la composition comprenant de l'eau et au moins un agent choisi dans le groupe constitué par des tensioactifs, des émulsifiants, des épaississants et des huiles.

9. Procédé topique non thérapeutiques de traitement de la peau et/ou du cuir chevelu, ledit procédé comprenant les étapes de mise en contact de la peau et/ou du cuir chevelu avec une composition cosmétique selon l'une quelconque des revendications 1 à 8.

10. Procédé selon la revendication 9 pour le maintien d'une homéostasie cutanée saine et/ou pour le maintien d'un équilibre du microbiome cutané.

11. Utilisation d'un mélange de 1,4-di(benzoxazol-2'-yl)benzène et au moins un composé choisi dans le groupe de l'hydroxyacétophénone, du 1,3-propanediol et de l'acide benzoïque ou d'un sel correspondant en tant que mélange d'agents antimicrobiens synergistes non thérapeutiques.

12. Utilisation selon la revendication 11, l'hydroxyacétophénone étant la p-hydroxyacétophénone et l'acide benzoïque ou un sel correspondant étant le benzoate de sodium.

13. Utilisation selon la revendication 11 ou 12, pour augmenter la durée de conservation et/ou la stabilité au stockage d'une composition topique.

14. Procédé d'inhibition ou de retardement d'une dégradation microbienne d'une composition topique, ledit procédé comprenant l'étape d'ajout à la composition cosmétique d'une quantité efficace de 1,4-di(benzoxazol-2'-yl)benzène et d'au moins un composé choisi dans le groupe de l'hydroxyacétophénone, du 1,3-propanediol et de l'acide benzoïque ou d'un sel correspondant.
